Europäisches Patentamt

European Patent Office

Office européen des brevets

(19)

(11) Numéro de publication : **0 378 942 B1**

(12) **FASCICULE DE BREVET EUROPEEN**

(45) Date de publication du fascicule du brevet :
**26.08.92 Bulletin 92/35**

(51) Int. Cl.$^5$ : **C07C 19/08, C07C 17/00**

(21) Numéro de dépôt : **89403551.8**

(22) Date de dépôt : **19.12.89**

(54) **Procédé de préparation du 1,1-dichloro-1-fluoroéthane.**

(30) Priorité : **27.12.88 US 290127**

(43) Date de publication de la demande :
**25.07.90 Bulletin 90/30**

(45) Mention de la délivrance du brevet :
**26.08.92 Bulletin 92/35**

(84) Etats contractants désignés :
**BE DE ES FR GB GR IT NL**

(56) Documents cités :
**FR-A- 2 365 542**
**JOURNAL OF FLUORINE CHEMISTRY, vol. 13,**
**1979, pages 7-18, Elsevier Sequoia, S.A., Lau-**
**sanne, CH; A.E. FEIRING: "Chemistry inhy-**
**drogen fluoride V. Catalysts for reaction of HF**
**with halogenated olefins"**

(73) Titulaire : **ELF ATOCHEM S.A.**
**4 & 8, Cours Michelet La Défense 10**
**F-92800 Puteaux (FR)**

(72) Inventeur : **Tung, Hsueh Sung**
**75 Brooklane Drive**
**Williamsville New York 14221 (US)**
Inventeur : **Smith, Addison Miles**
**80 Berryman Drive**
**Amherst New York 14226 (US)**

(74) Mandataire : **Leboulenger, Jean et al**
**ATOCHEM Département Propriété Industrielle**
**Cédex 42- La Défense 10**
**F-92091 Paris la Défense (FR)**

EP 0 378 942 B1

Jouve, 18, rue Saint-Denis, 75001 PARIS

EP 0 378 942 B1

## Description

La présente invention a pour objet un procédé pour la préparation du 1,1-dichloro-1-fluoroéthane et, plus particulièrement, un procédé de préparation du 1,1-dichloro-1-fluoroéthane utilisant un catalyseur à base de titane, d'hafnium ou de zirconium.

Le trichlorofluorométhane (connu dans le métier comme CFC-11) est actuellement disponible en quantités commerciales et est utilisé comme agent gonflant pour mousses de polyuréthane rigides. Le 1,1-dichloro-1-fluoroéthane (connu dans le métier comme HCFC-141b) est actuellement considéré comme un substitut du CFC-11 car le HCFC-141b n'attaque pas autant l'ozone stratosphérique que le CFC-11. Voir K.T. Dishart et al., POLYURETHANES WORLD CONGRESS 1987, 59 et G. Mouton et al., ibidem, 67. Puisque la demande en HCFC-141b s'accroîtra considérablement dans l'avenir, il existe un besoin pour des procédés, commercialement viables, de préparation du HCFC-141b.

Le HCFC-141b peut être produit par réaction du chlorure de vinylidène avec le fluorure d'hydrogène anhydre. La réaction s'exprime par l'équation suivante :

$$CH_2 = CCl_2 + HF \rightarrow CH_3CFCl_2$$

La réaction se déroule lentement à température ambiante, mais s'accélère aux températures supérieures à 40°C. A 60°C, cette réaction non-catalytique fournit la plus haute sélectivité en HCFC-141b.

Comme exemples de la réaction non-catalytique, A.L. Henne et al., J. Am. Chem. Soc. 65, 1271 (1943) rapportent qu'après trois heures à 65°C avec quatre moles de fluorure d'hydrogène, le chlorure de vinylidène donnait seulement 50 % de HCFC-141b, 15 % de goudron, 10 % de chlorure de vinylidène non transformé, 5 % de 1,1,1-trichloroéthane et des traces de 1-chloro-1,1-difluoroéthane (connu dans le métier comme HCFC-142b). Selon le brevet US 3 833 676, la réaction du 1,1,1-trichloroéthane avec le fluorure d'hydrogène en l'absence de catalyseur à 110°C pendant 15 minutes fournit 58,2 % de 1,1-dichloro-1-fluoroéthane, 41,7 % de 1-chloro-1,1-difluoroéthane, 0,10 % de 1,1,1-trichloroéthane et 0,03 % de 1,1,1-trifluoroéthane. En discontinu, une durée de plus de six heures est nécessaire pour convertir 99,9 % ou plus du chlorure de vinylidène. Une conversion élevée est souhaitable car il est difficile de séparer le chlorure de vinylidène de départ du HCFC-141b produit. Une augmentation de la température pour essayer d'accroître le rendement en HCFC-141b ne fait qu'augmenter la formation d'un sous-produit, le HCFC-142b, et donc diminue la sélectivité en HCFC-141b.

Les longues durées de réaction requises pour obtenir un bon rendement en HCFC-141b rendent le procédé impraticable industriellement et différents catalyseurs ont été proposés.

La réaction du chlorure de vinylidène et du fluorure d'hydrogène anhydre en présence de pentafluorure de tantale est décrite dans le brevet US 4 258 225. La réaction est effectuée à 25°C pendant trois heures. Le rendement en HCFC-141b est d'environ 36 % et le produit restant est une huile foncée. Le faible rendement en HCFC-141b fait que ce procédé n'est pas praticable commercialement.

Dans J. of Fluorine Chem. 13, 7 (1979), A.E. Feiring mentionne l'addition de fluorure d'hydrogène sur le tétrachloroéthylène, le trichloroéthylène et des composés analogues en présence de pentafluorure de tantale, de pentafluorure de niobium, de tétrachlorure de titane ou de pentachlorure de molybdène. L'article conclut que le pentafluorure de tantale apparaît être le plus actif des matériaux testés. Il rapporte que la réaction de 0,4 mole de chlorure de vinylidène et 0,5 mole de fluorure d'hydrogène anhydre en présence de 0,01 mole de pentafluorure de tantale à 25°C pendant trois heures fournit seulement 40 % de HCFC-141b, le reste étant des goudrons. Ce procédé n'est pas attractif commercialement parce que l'élimination des goudrons est onéreuse et qu'un faible rendement en HCFC-141b est inacceptable.

Le brevet US 2 005 708 enseigne la préparation de chlorofluoroéthanes, mais pas celle du 1,1-dichloro-1-fluoroéthane, par réaction d'éthanes ou d'éthènes chlorés avec le fluorure d'hydrogène en présence d'un halogénure d'antimoine à des températures supérieures à 95°C, sous pression.

Le brevet US 2 452 975 enseigne la préparation de chlorofluoroéthanes, mais pas celle du 1,1-dichloro-1-fluoroéthane, par réaction de chloroéthanes avec le fluorure d'hydrogène en présence de chlorure stannique.

La préparation de fluorocarbures, mais pas celle du 1,1-dichloro-1-fluoroéthane, par réaction d'hydrocarbures chlorés avec l'acide fluorhydrique en présence de fluorure d'aluminium, de chrome ou de nickel comme catalyseur à une température de 275 à 425°C fait l'objet du brevet US 4 147 733.

Selon le brevet US 2 495 407, la réaction du dichloroéthylène et du fluorure d'hydrogène anhydre en présence de chlorure stannique pendant 1,75 heure donne du 1,1,-dichloro-1-fluoroéthane avec une conversion de 32,7 % et un rendement de 43 % par rapport au dichloroéthylène. Le faible rendement en HCFC-141b rend ce procédé commercialement inattractif.

Le brevet US 4 766 258 enseigne que la réaction du chlorure de vinylidène et du fluorure d'hydrogène anhydre en présence d'un catalyseur halogénure stannique et d'eau pendant une heure donne un rendement de 29 % en HCFC-141b. Il y est aussi indiqué que, si on utilise un autre halogénure qu'un halogénure stannique,

2

par exemple le tétrachlorure de titane, pour produire un fluoro-hydrocarbure par réaction du fluorure d'hydrogène anhydre et d'un halogéno-hydrocarbure contenant de l'hydrogène, il se forme des sous-produits tels que des produits lourds, des oligomères et des précipités noirs, en quantités encore plus grandes que si la réaction est menée avec un halogénure stannique comme catalyseur. Là encore, un faible rendement en HCFC-141b est indésirable.

Il existe donc dans ce domaine un besoin pour un procédé de préparation du 1,1-dichloro-1-fluoroéthane dont la durée de réaction serait plus courte que celles des procédés connus et qui permettrait de produire le 1,1-dichloro-1-fluoroéthane avec un rendement supérieur à celui des procédés connus.

La présente invention répond à ce besoin en proposant un procédé de préparation du 1,1-dichloro-1-fluoroéthane qui comprend l'étape consistant à faire réagir du chlorure de vinylidène avec un excès de fluorure d'hydrogène anhydre en présence d'une quantité catalytique d'un catalyseur de formule :

$$R_mAX_nF_p$$

pendant une durée et à une température suffisantes pour former du 1,1-dichloro-1-fluoroéthane.

Dans la formule ci-dessus, A désigne le titane, le zirconium ou l'hafnium, R représente un radical alkyle, alcényle ou aryle, X désigne le chlore ou le brome, m est un nombre allant de 0 à 2, n un nombre allant de 0 à 4 et p un nombre allant de 0 à 4, mais l'un au moins des nombres n et p n'est pas nul et la somme m+n+p satisfait la valence de A.

L'expression "excès de fluorure d'hydrogène anhydre" telle qu'utilisée ici signifie que le rapport molaire du fluorure d'hydrogène anhydre au chlorure de vinylidène est supérieur à 1:1. Autrement dit, on met en oeuvre un défaut de chlorure de vinylidène.

Par rapport à la réaction non catalysée du chlorure de vinylidène et du fluorure d'hydrogène anhydre qui fait l'objet de l'article de Henne et du brevet US 3 833 676 précités, le procédé selon la présente invention est plus avantageux puisqu'il réduit de plus de 75 % la durée de réaction pour obtenir une conversion de 99,9 % du chlorure de vinylidène. Comparé aux procédés du brevet US 4 258 225 et de l'article de Feiring précités qui utilisent du pentafluorure de tantale, le procédé selon la présente invention donne en une durée plus courte un meilleur rendement en 1,1-dichloro-1-fluoroéthane ; il en est de même vis-à-vis du procédé selon le brevet US 2 495 407 qui emploie du chlorure stannique pour catalyser la réaction du dichloroéthylène et du fluorure d'hydrogène anhydre.

Bien que le brevet US 4 766 258 mentionne l'emploi du tétrachlorure de titane dans la fluoration d'halogéno-hydrocarbures contenant de l'hydrogène, il ne décrit pas le procédé selon la présente invention. De plus, puisque le brevet US 4 766 258 indique des rendements inférieurs à 29 % lors de l'emploi de tétrachlorure de titane pour fluorer des halogénohydrocarbures contenant de l'hydrogène, ce brevet n'aurait pas conduit l'homme du métier au procédé de la présente invention.

Bien que l'article de Feiring susmentionné enseigne l'emploi du tétrachlorure de titane dans la fluoration du tétrachloroéthylène, il ne décrit pas le procédé selon l'invention. Puisqu'il conclut que le pentafluorure de tantale est le catalyseur le plus actif et rapporte que la réaction de 0,4 mole de chlorure de vinylidène et 0,5 mole de fluorure d'hydrogène anhydre en présence de pentafluorure de tantale à 25°C pendant 3 heures donne seulement 40 % de HCFC-141b, cet article n'aurait pas conduit l'homme du métier au procédé selon la présente invention.

Le fluorure d'hydrogène anhydre et le chlorure de vinylidène, commercialement disponibles, peuvent être utilisés dans le procédé selon l'invention. L'addition du fluorure d'hydrogène anhydre à la double liaison carbone-carbone du chlorure de vinylidène est une réaction stoechiométrique nécessitant une mole de fluorure d'hydrogène anhydre par mole de chlorure de vinylidène utilisée. En général, le fluorure d'hydrogène anhydre et le chlorure de vinylidène sont mis à réagir avec un excès de fluorure d'hydrogène anhydre par rapport au chlorure de vinylidène. Il a été trouvé que, puisque les goudrons ou produits lourds indésirables sont principalement formés par des réactions de couplage ou de polymérisation de molécules de chlorure de vinylidène, la quantité de goudrons ou de produits lourds formés pendant la réaction peut être minimisée en minimisant la quantité de chlorure de vinylidène de départ. Ainsi, l'emploi d'un excès de chlorure de vinylidène est désavantageux. De préférence, le fluorure d'hydrogène anhydre et le chlorure de vinylidène sont mis à réagir dans un rapport molaire d'environ 1,5:1 à environ 5:1. L'emploi de fluorure d'hydrogène anhydre en une quantité supérieure au rapport molaire 5:1 provoque la formation d'autres alcanes fluorés. Plus préférentiellement, le fluorure d'hydrogène anhydre et le chlorure de vinylidène sont mis à réagir dans un rapport molaire d'environ 1,5:1 à environ 4:1 et, plus particulièrement, d'environ 1,5:1 à environ 3:1.

En ce qui concerne le symbole R de la formule du catalyseur, on peut mentionner comme exemples de radicaux alkyle les radicaux méthyle, éthyle, isopropyle, butyle, pentyle, hexyle, heptyle et octyle. Comme exemples de radicaux aryle on peut mentionner les radicaux phényle et naphtyle ; le radical aryle peut aussi être substitué et donc inclure des radicaux tels que benzyle, tolyle et halogéno-phényle. Des exemples de radicaux alcényle sont le radical cyclopentadiényle et le radical pentaméthylcyclopentadiényle.

Quand A est le zirconium, les catalyseurs préférés sont le tétrachlorure de zirconium, le tétrafluorure de zirconium, le tétrabromure de zirconium et le dichlorure de zirconocène. Quand A est l'hafnium, les catalyseurs préférés sont le tétrachlorure d'hafnium, le tétrafluorure d'hafnium et le dichlorure d'hafnocène. Quand A est le titane, les catalyseurs préférés comprennent le trichlorure de titane, le tétrachlorure de titane, le trifluorure de titane, le tétrafluorure de titane, le tétrabromure de titane et le dichlorure de titanocène. Le catalyseur le plus préféré est le tétrachlorure de titane. Les formes commerciales de tétrachlorure de zirconium, dichlorure de zirconocène, tétrachlorure d'hafnium, dichlorure d'hafnocène, tétrachlorure de titane, tétrabromure de titane et dichlorure de titanocène peuvent être utilisées pour la mise en oeuvre du procédé selon la présente invention.

On utilise une quantité catalytique de catalyseur. En général, cette quantité est d'environ 0,01 à environ 50 moles de catalyseur pour 100 moles de chlorure de vinylidène utilisé. De préférence, la quantité de catalyseur utilisée pour 100 moles de chlorure de vinylidène est d'environ 0,1 à environ 10 moles, avantageusement d'environ 0,2 à environ 5 moles et encore mieux d'environ 0,3 à environ 2 moles.

La réaction est de préférence menée à une température allant d'environ 30 à environ 100°C. Aux températures inférieures à 30°C, la réaction devient trop lente pour être utile. Aux températures supérieures à 100°C, la formation de sous-produits diminue le rendement en HCFC-141b. Il est particulièrement avantageux d'opérer à une température allant d'environ 50 à environ 65°C, domaine correspondant à la meilleure sélectivité en HCFC-141b.

La réaction peut être menée à la pression atmosphérique, mais on opère de préférence sous une pression allant d'environ 10 à environ 130 psig (69 à 897 kPa). Un dégazage partiel du réacteur, de façon à éviter un accroissement de pression dû à la formation de chlorure d'hydrogène, permet de minimiser avantageusement la formation de sous-produits.

Le réacteur est utilement construit avec des matériaux résistant à l'action du fluorure d'hydrogène anhydre, par exemple des matériaux métalliques ou polymériques. Pour les réactions menées à des températures supérieures au point d'ébullition du chlorure de vinylidène (environ 30°C), on utilise un réacteur fermé de façon à minimiser la perte de chlorure de vinylidène et de fluorure d'hydrogène anhydre.

D'une façon générale, le chlorure de vinylidène, le fluorure d'hydrogène anhydre et le catalyseur sont introduits dans le réacteur dans un ordre quelconque, sous réserve d'utiliser un excès de fluorure d'hydrogène anhydre par rapport au chlorure de vinylidène. Le contenu du réacteur est porté à la température appropriée et agité pendant une durée suffisante pour que le fluorure d'hydrogène anhydre et le chlorure de vinylidène réagissent. L'avancement de la réaction peut être suivi par des prélèvements périodiques d'échantillons. La durée de réaction est de préférence inférieure à environ 2 heures et, plus particulièrement, comprise entre environ 0,1 et environ 1,5 heure.

Selon une modalité préférée, on dissout une quantité catalytique de catalyseur dans le fluorure d'hydrogène anhydre et on chauffe ce mélange jusqu'à une température d'environ 50 à environ 65°C avant d'y ajouter le chlorure de vinylidène. Le fluorure d'hydrogène anhydre a une tension de vapeur d'environ 40 psig (276 kPa) à 60°C et un point d'ébullition de 19,5°C ; à la pression atmosphérique, le point d'ébullition du tétrachlorure de titane est de 136,4 °C. Quand une quantité catalytique de tétrachlorure de titane est mélangée avec du fluorure d'hydrogène anhydre à 60°C, le mélange a une tension de vapeur d'environ 65-70 psig (448-483 kPa). Cet accroissement de pression laisse penser que le fluorure d'hydrogène anhydre a réagi avec le tétrachlorure de titane pour former un composé à bas point d'ébullition tel que HCl. On pense qu'il s'est aussi formé plusieurs composés de titane et l'équation suivante résume la réaction probable :

$$4\ TiCl_4\ +\ 10HF \rightarrow 10HCl\ +\ TiCl_3F\ +\ TiCl_2F_2\ +\ TiClF_3\ +\ TiF_4$$

On suppose donc que le tétrachlorure de titane peut être complètement fluoré en tétrafluorure de titane ou partiellement fluoré en composés tels que $TiCl_3F$, $TiCl_2F_2$ et $TiClF_3$. Le mélange de tétrachlorure de titane et de fluorure d'hydrogène anhydre pourrait contenir toute combinaison de ces quatre composés fluorés du titane. On pense que la réaction entre le chlorure de vinylidène et le fluorure d'hydrogène anhydre peut être catalysée par n'importe lequel des cinq composés du titane ou n'importe laquelle de leurs combinaisons. Ainsi, le catalyseur de formule $R_mAX_nF_p$ tel que défini précédemment couvre $TiCl_3F$, $TiCl_2F_2$ et $TiClF_3$.

Le 1,1-dichloro-1-fluoroéthane produit peut être isolé par n'importe laquelle des diverses techniques connues. Le contenu du réacteur peut être versé sur de la glace et la couche organique récupérée, lavée à l'eau et séchée avec un dessicant. Le produit peut être analysé par les techniques habituelles telles que la chromatographie gaz-liquide, la spectroscopie RMN et la spectrométrie de masse.

Le procédé selon la présente invention peut être conduit en mode discontinu ou en mode continu.

Le 1,1-dichloro-1-fluoroéthane est particulièrement utile en tant qu'agent gonflant pour la production de mousses de polyuréthane rigides thermoisolantes. Voir par exemple les brevets US 4 652 589, 4 686 240, 4 699 932, 4 701 474, 4 717 518 et 4 727 094.

Les exemples suivants illustrent l'invention sans la limiter.

Pour chaque Exemple et chaque Comparaison, le rapport molaire du fluorure d'hydrogène anhydre au chlorure de vinylidène était d'environ 1,9:1. Chaque essai a été effectué sous agitation à 2400 tours par minute.

Dans le tableau I suivant, les abréviations "Cat", "VC", "140a" et "HB" désignent respectivement le catalyseur, le chlorure de vinylidène, le 1,1,1-trichloroéthane et les composés lourds. Dans la colonne "Total HB", le nombre placé entre parenthèses indique le nombre de composés lourds observés.

## COMPARAISON 1

200 g de fluorure d'hydrogène anhydre ont été chargés dans un autoclave de 1 litre à la température ambiante, puis l'autoclave a été chauffé jusqu'à la température de réaction, 60°C, sous agitation. Ensuite, 500 g de chlorure de vinylidène ont été pompés dans l'autoclave à débit constant. L'évolution de la réaction a été suivi par le prélèvement périodique d'un échantillon de la couche organique, au moyen d'un tube plongeur installé dans le réacteur. L'échantillon était immédiatement refroidi avec un mélange de glace et d'eau, puis la couche organique était isolée, séchée et analysée sur un chromatographe à gaz. La réaction était considérée comme terminée quand 99,9 % de chlorure de vinylidène a été converti.

La réaction terminée, le réacteur a été refroidi rapidement jusqu'à température ambiante, puis la couche organique a été récupérée et lavée avec un mélange de glace et d'eau. Le produit brut lavé a ensuite été isolé, séché et analysé. Les résultats figurent à la ligne C1 du tableau I suivant, le tableau II donnant la pression exprimée en unités métriques.

## TABLEAU I

| EX | CAT. | TEMP. (°C) | VC DEBIT D'ALIM. (g/min) | % MOL FRAC. (Cat/ VC) | PRESSION (psig) | DUREE (heures) | ANALYSE DU PRODUIT (%) | | | | |
|----|------|------------|--------------------------|-----------------------|-----------------|----------------|------|------|------|------|-----------|
| | | | | | | | 142b | 141b | VC | 140a | Total HB |
| C1 | Néant | 60 | 2,16 | – | 80-120 | 6,1 | 1,3 | 93 | 0,10 | 4,2 | 1,2 (3) |
| C2 | $BF_3$ | 60 | 8,64 | 0,78 | 100-120 | 1,5 | 0,9 | 91 | 0,03 | 2,9 | 4,8 (5) |
| C3 | $BF_3$ | 49 | 8,08 | 1,23 | 75-80 | 2,0 | 0,3 | 87 | 0,15 | 2,7 | 10,2 (5) |
| C4 | $TaF_5$ | 60 | 8,64 | 0,76 | 100-120 | 1,25 | 0,9 | 94 | 0,00 | 2,3 | 2,2 (4) |
| C5 | $TaF_5$ | 60 | 9,09 | 0,38 | 100-120 | 1,5 | 0,6 | 94 | 0,03 | 2,4 | 3,2 (4) |
| C6 | $SnCl_4$ | 60 | 8,96 | 0,81 | 110-150 | 1,5 | 4,4 | 84 | 1,60 | 9,9 | 0,4 (1) |
| 1 | $TiCl_4$ | 60 | 10,20 | 0,83 | 120-130 | 1,25 | 0,4 | 96 | 0,10 | 1,6 | 1,6 (2) |
| 2 | $TiCl_4$ | 60 | 9,43 | 0,90 | 124-126 | 1,25 | 0,4 | 96 | 0,09 | 1,9 | 0,8 (3) |
| 3 | $TiCl_4$ | 50 | 8,77 | 0,97 | 87-95 | 1,50 | 0,4 | 97 | 0,20 | 1,4 | 1,1 (3) |
| 4 | $TiCl_4$ | 60 | 10,87 | 0,92 | 102-113 | 1,25 | 0,7 | 97 | 0,10 | 1,0 | 1,3 (3) |

EP 0 378 942 B1

## TABLEAU II

| EX. | PRESSION (kPa) |
|-----|----------------|
| C1 | 552-828 |
| C2 | 690-828 |
| C3 | 518-552 |
| C4 | 690-828 |
| C5 | 690-828 |
| C6 | 759-1035 |
| 1 | 828-897 |
| 2 | 856-869 |
| 3 | 600-656 |
| 4 | 704-780 |

Les principaux sous-produits de la réaction comprennent du HCFC-142b, du HCC-140a, du HCl et des produits lourds. Ces produits lourds sont constitués par des composés fluorés à 4 et 6 atomes de carbone, certains polymères et le stabilisant présent dans le chlorure de vinylidène de départ. La composition des produits bruts a été déterminée par analyse du produit récupéré en phase liquide. Le HCFC-142b a un point d'ébullition beaucoup plus bas que la température ambiante et par conséquent la sélectivité réelle en HCFC-142b est légèrement supérieure à celle indiquée dans le tableau I. Le HCC-140a a été formé par réaction entre le chlorure de vinylidène et le chlorure d'hydrogène. Ce dernier et le HCFC-142b ont été formés par réaction entre le HCFC-141b et HF. Les produits lourds proviennent essentiellement de réactions de couplage et de polymérisation de molécules de chlorure de vinylidène.

Il ressort des résultats que la réaction non catalysée requiert une longue durée (6,1 heures).

**COMPARAISONS 2 ET 3**

Dans le même autoclave que pour la Comparaison 1, deux essais séparés ont été effectués dans des conditions semblables. Après avoir chargé les 200 g de fluorure d'hydrogène anhydre, du trifluorure de bore a été introduit dans le réacteur. Le mélange a ensuite été chauffé jusqu'à 60 et 49°C respectivement, avant de procéder à l'alimentation du chlorure de vinylidène (500 g). On a opéré de la même manière qu'à la comparaison 1 pour suivre le déroulement de la réaction, récupérer le produit brut et l'analyser. Les résultats figurent aux lignes C2 et C3 du tableau I.

Le trifluorure de bore semble provoquer des réactions de couplage et fournit plus de produits lourds (ou goudrons). Il en résulte une sélectivité en HCFC-141b inférieure à celle de la réaction non catalytique de la Comparaison 1.

**COMPARAISONS 4 ET 5**

Ces deux essais ont été réalisés dans le même appareillage qu'à la Comparaison 1 et sous les mêmes conditions réactionnelles qu'à la Comparaison 2, mais en utilisant le pentafluorure de tantale comme catalyseur. Les résultats figurent aux lignes C4 et C5 du tableau I.

Ces résultats sont surprenants eu égard à ceux rapportés dans la référence Feiring précitée. Selon cet article, la réaction de 0,4 mole de chlorure de vinylidène et 0,5 mole de fluorure d'hydrogène anhydre en présence de pentafluorure de tantale à 25°C pendant 3 heures donnait seulement 40 % de HCFC-141b. On suppose que le meilleur rendement provient de la dissolution du pentafluorure de tantale dans le fluorure d'hydrogène anhydre et du chauffage de ce mélange jusqu'à la température réactionnelle avant d'y ajouter un défaut de chlorure de vinylidène.

Le pentafluorure de tantale améliore la sélectivité et réduit la durée de réaction, mais produit plus de goudrons qu'en absence de catalyseur.

## COMPARAISON 6

Le tétrachlorure d'étain a été étudié dans cet essai où le nombre de moles de catalyseur était à peu près le même que dans la Comparaison 2 et les autres conditions opératoires étaient aussi tout-à-fait similaires. Le produit brut a été récupéré aussi de la même façon. On a observé une troisième phase plus lourde que la phase organique, ce qui suggère que le tétrachlorure d'étain n'est pas très soluble dans le fluorure d'hydrogène anhydre ou dans la phase organique. Les résultats de l'analyse du produit organique brut figurent à la ligne C6 du tableau I.

Le tétrachlorure d'étain réduit la quantité de composés lourds, mais ne catalyse pas la réaction aussi bien que les autres catalyseurs. Il favorise la formation de HCFC-142b et de HCC-140a, d'où une moindre sélectivité en HCFC-141b.

## EXEMPLES 1-3

L'activité catalytique du tétrachlorure de titane a aussi été étudiée dans le même autoclave de 1 litre. On a opéré dans des conditions réactionnelles semblables à celles des Comparaisons 4 et 5, mais en utilisant comme catalyseur le tétrachlorure de titane au lieu du pentafluorure de tantale. Chacun des trois essais a été effectué avec du tétrachlorure de titane frais. Les exemples 1 et 2 ont été réalisés à 60°C et l'exemple 3 à 50°C. Les résultats figurent aux lignes 1, 2 et 3 du tableau I précédent.

Avec le pentafluorure de tantale, le rendement en 141b était de 94 %. Il est de 96-97 % dans le procédé revendiqué. Ainsi, le tétrachlorure de titane est plus sélectif et produit moins de goudrons ; il est en outre moins onéreux que le pentafluorure de tantale.

## EXEMPLE 4

Les conditions opératoires pour cet exemple étaient presque identiques à celles de l'exemple 3, sauf que le tétrachlorure de titane et environ 90 g de fluorure d'hydrogène anhydre étaient recyclés de l'exemple 3. Par ailleurs, 105 g de fluorure d'hydrogène anhydre frais étaient ajoutés pour la réaction en estimant que le catalyseur était retenu à 95 % dans la phase de fluorure d'hydrogène anhydre.

Les résultats de cet essai figurent à la ligne 4 du tableau I précédent. Le tétrachlorure de titane recyclé montre la même activité catalytique que le catalyseur frais utilisé dans les exemples 1 et 2.

Il ressort des exemples 1 à 4 que le tétrachlorure de titane donne la meilleure sélectivité en 1,1-dichloro-1-fluoroéthane et ne produit pas plus de composés lourds que la réaction non-catalytique.

## EXEMPLES 5-15

Les catalyseurs du tableau III suivant ont été mis en oeuvre dans le même appareillage et selon la même procédure qu'à la Comparaison 2, dans les conditions suivantes :
– température : 60°C
– débit d'alimentation du chlorure de vinylidène : 10 g/mn
– catalyseur/chlorure de vinylidène (% molaire) : 0,8-1
– pression : 552-828 kPa (80-120 psig)
– durée : 1,5 heure

## TABLEAU III

| EXEMPLE | CATALYSEUR |
|---|---|
| 5 | $ZrCl_4$ |
| 6 | $ZrF_4$ |
| 7 | $ZrCl_2(C_5H_5)_2$ |
| 8 | $HfCl_4$ |
| 9 | $HfF_4$ |
| 10 | $HfCl_2(C_5H_5)_2$ |
| 11 | $TiCl_3$ |
| 12 | $TiCl_4$ |
| 13 | $TiF_3$ |
| 14 | $TiF_4$ |
| 15 | $TiCl_2(C_5H_5)_2$ |

**Revendications**

1.  Procédé pour la préparation d'un hydrochlorofluorocarbure, caractérisé en ce qu'il comprend une étape consistant à faire réagir du chlorure de vinylidène avec un excès de fluorure d'hydrogène anhydre en présence d'une quantité catalytique d'un catalyseur de formule :

$$R_mAX_nF_p$$

dans laquelle A désigne le titane, le zirconium ou l'hafnium, R représente un radical alkyle, alcényle ou aryle, X désigne le chlore ou le brome, m est un nombre allant de 0 à 2, n un nombre allant de 0 à 4 et p un nombre allant de 0 à 4, mais l'un au moins des nombres n et p n'est pas nul et la somme m + n + p satisfait la valence de A, pendant une durée et à une température suffisantes pour former du 1,1-dichloro-1-fluoroéthane.

2.  Procédé selon la revendication, dans lequel le rapport molaire du fluorure d'hydrogène anhydre au chlorure de vinylidène est d'environ 1,5:1 à environ 5:1.

3.  Procédé selon la revendication 1, dans lequel le fluorure d'hydrogène anhydre et le chlorure de vinylidène sont mis à réagir dans un rapport molaire allant d'environ 1,5:1 à environ 4:1.

4.  Procédé selon la revendication 1, dans lequel le fluorure d'hydrogène anhydre et le chlorure de vinylidène sont mis à réagir dans un rapport molaire allant d'environ 1,5:1 à environ 3:1.

5.  Procédé selon l'une des revendications 1 à 4, dans lequel la réaction est conduite à une température allant d'environ 30 à environ 100°C.

6.  Procédé selon l'une des revendications 1 à 4, dans lequel la réaction est menée à une température allant d'environ 50 à environ 65°C.

7.  Procédé selon l'une des revendications 1 à 4, dans lequel avant réaction avec le chlorure de vinylidène, le catalyseur est dissous dans le fluorure d'hydrogène anhydre et chauffé à une température allant d'environ 50 à environ 65°C.

8.  Procédé selon l'une des revendications 1 à 7, dans lequel le catalyseur est utilisé en une quantité allant d'environ 0,3 à environ 2 moles pour cent moles de chlorure de vinylidène.

EP 0 378 942 B1

9. Procédé selon l'une des revendications 1 à 8, dans lequel le catalyseur est le tétrachlorure de zirconium, le dichlorure de zirconocène, le tétrachlorure d'hafnium, le dichlorure d'hafnocène, le tétrachlorure de titane, le dichlorure de titanocène ou le tétrabromure de titane.

**Patentansprüche**

1. Verfahren zur Herstellung eines Chlorfluorkohlenwasserstoffs, dadurch gekennzeichnet, daß in einer Stufe Vinylidenchlorid mit überschüssigem wasserfreiem Fluorwasserstoff in Gegenwart einer katalytischen Menge eines Katalysators der Formel
$$R_mAX_nF_p,$$
in der A Titan, Zirkonium oder Hafnium, R einen Alkyl-, Alkenyl- oder Arylrest, X Chlor oder Brom, m eine ganze Zahl von 0 bis 2, n eine ganze Zahl von 0 bis 4 und p eine ganze Zahl von 0 bis 4 bedeuten, wobei mindestens einer der Werte n und p nicht Null ist und die Summe m + n + p der Wertigkeit von A entspricht, während einer Zeitdauer und bei einer temperatur, die zur Bildung von 1,1-Dichlor-1-fluorethan ausreichen, umgesetzt wird.

2. Verfahren nach Anspruch 1, bei dem das Molverhältnis vom wasserfreien Fluorwasserstoff zum Vinylidenchlorid von ungefähr 1,5:1 bis ungefähr 5:1 beträgt.

3. Verfahren nach Anspruch 1, bei dem der wasserfreie Fluorwasserstoff und das Vinylidenchlorid bei einem Molverhältnis von ungefähr 1,5:1 bis ungefähr 4:1 umgesetzt werden.

4. Verfahren nach Anspruch 1, bei dem der wasserfreie Fluorwasserstoff und das Vinylidenchlorid bei einem Molverhältnis von ungefähr 1,5:1 bis ungefähr 3:1 umgesetzt werden.

5. Verfahren nach einem der Ansprüche 1 bis 4, in dem die Umsetzung bei einer Temperatur von ungefähr 30 bis ungefähr 100°C durchgeführt wird.

6. Verfahren nach einem der Ansprüche 1 bis 4, in dem die Umsetzung bei einer Temperatur von ungefähr 50 bis ungefähr 65°C durchgeführt wird.

7. Verfahren nach einem der Ansprüche 1 bis 4, bei dem vor der Umsetzung mit dem Vinylidenchlorid der Katalysator im wasserfreien Fluorwasserstoff gelöst und auf eine Temperatur von ungefähr 50 bis ungefähr 65°C erhitzt wird.

8. Verfahren nach einem der Ansprüche 1 bis 7, bei dem der Katalysator in einer Menge von ungefähr 0,3 bis ungefähr 2 Molen pro 100 Molen Vinylidenchlorid verwendet wird.

9. Verfahren nach einem der Ansprüche 1 bis 8, bei dem der Katalysator Zirkoniumtetrachlorid, Zirkonocendichlorid, Hafniumtetrachlorid, Hafnocendichlorid, Titantetrachlorid, Titanocendichlorid oder titantetrabromid ist.

**Claims**

1. Process for the preparation of a chlorofluorohydrocarbon, characterised in that it comprises a stage consisting in reacting vinylidene chloride with an excess of anhydrous hydrogen fluoride in the presence of a catalytic quantity of a catalyst of formula:
$$R_mAX_nF_p$$
in which A denotes titanium, zirconium or hafnium, R denotes an alkyl, alkenyl or aryl radical, X denotes chlorine or bromine, m is a number ranging from 0 to 2, n a number ranging from 0 to 4 and p a number ranging from 0 to 4, but at least one of the numbers n and p is not zero and the sum m + n + p satisfies the valency of A, for a time and at a temperature which are sufficient to form 1,1-dichloro-1-fluoroethane.

2. Process according to Claim 1, in which the molar ratio of anhydrous hydrogen fluoride to vinylidene chloride is from approximately 1.5:1 to approximately 5:1.

3. Process according to Claim 1, in which anhydrous hydrogen fluoride and vinylidene chloride are reacted

10

in a molar ratio ranging from approximately 1.5:1 to approximately 4:1.

4. Process according to Claim 1, in which anhydrous hydrogen fluoride and vinylidene chloride are reacted in a molar ratio ranging from approximately 1.5:1 to approximately 3:1.

5. Process according to one of Claims 1 to 4, in which the reaction is conducted at a temperature ranging from approximately 30 to approximately 100°C.

6. Process according to one of Claims 1 to 4, in which the reaction is conducted at a temperature ranging from approximately 50 to approximately 65°C.

7. Process according to one of Claims 1 to 4, in which, before reaction with vinylidene chloride, the catalyst is dissolved in anhydrous hydrogen fluoride and heated to a temperature ranging from approximately 50 to approcimately 65°C.

8. Process according to one of Claims 1 to 7, in which the catalyst is employed in a quantity ranging from approximately 0.3 to approximately 2 moles per hundred moles of vinylidene chloride.

9. Process according to one of Claims 1 to 8, in which the catalyst is zirconium tetrachloride, zirconocene dichloride, hafnium tetrachloride, hafnocene dichloride, titanium tetrachloride, titanocene dichloride and titanium tetrabromide.